# EUROPEAN PATENT APPLICATION

(11) **EP 3 466 468 A1**
(43) Date of publication of application: **10.04.2019**
(21) Application number: 17806871.4
(22) Date of filing: 27.02.2017
(51) Int. Cl.: A61M 5/32, A61M 5/31

(54) **OUTER CAP OF MEDICINAL FLUID INJECTION INSTRUMENT**

(30) Priority: 02.06.2016 KR 20160068664
(71) Applicant: Ra, Yong-Kuk, Gumi-si, Gyeongsangbuk-do 39346 (KR)
(72) Inventor: Ra, Yong-Kuk, Gumi-si, Gyeongsangbuk-do 39346 (KR)
(74) Representative: Wilson Gunn
(86) International application number: PCT/KR2017/002114
(87) International publication number: WO 2017/209378

(57) **Abstract**

The present invention relates to an outer cap of a liquid medicine-injecting device, and more particularly, to an apparatus in which an outer cap configured to temporarily form a suction flow passage only upon suction of a liquid medicine and to be removed for opening an injection flow passage upon injection of the liquid medicine is improved in a liquid medicine-injecting device including a filter for filtering foreign substances contained in a liquid medicine, so that firm sealing is established between an inner peripheral surface of the outer cap and a peripheral surface of the liquid medicine-injecting device so as to reliably generate a negative pressure in the injection flow passage and particularly to prevent the liquid medicine from penetrating an injection needle upon suction of the liquid medicine. An outer cap 100 of a liquid medicine-injecting device 200 according to the present invention includes a suction needle 110 for sucking a liquid medicine and a cap hub 120 hermetically coupled to the liquid medicine-injecting device 200, and the outer cap 100 has an internal space extending from the suction needle 110 to the cap hub 120, wherein the internal space is formed with an injection needle-receiving portion 130 for receiving an injection needle 210 when the liquid medicine-injecting device 200 is assembled; and wherein a sealing means configured to be in contact with a peripheral surface of the liquid medicine-injecting device 200 so as to maintain airtightness between the inner space and the injection needle-receiving portion 130 when the liquid medicine-injecting device 200 is assembled is provided at an inlet of the injection needle-receiving portion 130. The outer cap of the liquid medicine-injecting device allows the liquid medicine-injecting device having the filter to more firmly suck the liquid medicine, and particularly fundamentally prevents the injection needle from being contaminated by a liquid medicine that has not yet undergone filtration, to maximize marketability and user's safety of the liquid medicine-injecting device having the filter.

## Description

### [Technical Field]

The present invention relates to an outer cap of a liquid medicine-injecting device, and more particularly, to an apparatus in which an outer cap configured to temporarily form a suction flow passage only upon suction of a liquid medicine and to be removed for opening an injection flow passage upon injection of the liquid medicine is improved in a liquid medicine-injecting device including a filter for filtering foreign substances contained in a liquid medicine, so that firm sealing is established between an inner peripheral surface of the outer cap and a peripheral surface of the liquid medicine-injecting device so as to reliably generate a negative pressure in the injection flow passage and particularly to prevent the liquid medicine from penetrating an injection needle upon suction of the liquid medicine, thereby allowing the liquid medicine-injecting device having the filter to more firmly suck the liquid medicine, and particularly fundamentally preventing the injection needle from being contaminated by a liquid medicine that has not yet undergone filtration, to maximize marketability and user's safety of the liquid medicine-injecting device having the filter.

### [Background Art]

In general, a syringe is an instrument for injecting a liquid medicine into a body of an animal/plant and is configured to pierce a skin with a sharp tip thereof to allow the liquid medicine to be injected into any tissue of the body.

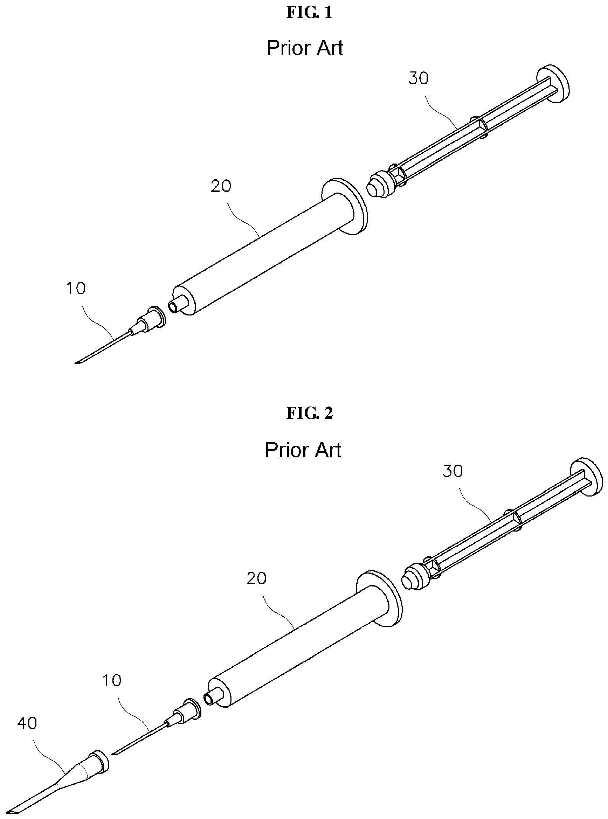

Fig. 1 is an exploded perspective view illustrating a conventional syringe. As shown in Fig. 1, the syringe generally includes a cylinder 20 to which an injection needle 10 is coupled and in which a liquid medicine is contained, and a plunger 30 provided in the cylinder 20 so as to be movable forward and backward.

In this conventional syringe, as the plunger 30 is moved backward, a negative pressure is generated in the cylinder 20 and the cylinder is then filled with a liquid medicine. As the plunger is moved forward, the liquid medicine in the cylinder 20 is discharged through the injection needle 10 by a positive pressure and then injected into a patient's body.

However, when this conventional syringe is used, there is concern that foreign substances incorporated in the liquid medicine itself or foreign substances such as glass particles scattered into and mixed with the liquid medicine during a process of breaking and opening an ampoule in which the liquid medicine is stored may be injected together with the liquid medicine into the patient's body.

To solve this problem, a filtering syringe provided with a filter for filtering foreign substances contained in a liquid medicine has been developed.

First, as disclosed in Korean Patent Laid-Open Publication No. 2012-87587, a conventional filtering syringe in which a filter for filtering foreign substances is provided in an injection needle or a cylinder performs a function of filtering foreign substances from a liquid medicine sucked into the cylinder.

However, since foreign substances had been stuck to an outer surface of the injection needle introduced into an ampoule when the liquid medicine is sucked, or foreign substances incorporated in the liquid medicine remaining within the injection needle had not been filtered, it was impossible to fundamentally prevent the foreign substances from being injected together with the liquid medicine into a patient's body.

In response thereto, US Patent No. 4,180,071 discloses an example in which a filter for filtering foreign substances is provided in a cap for covering an injection needle.

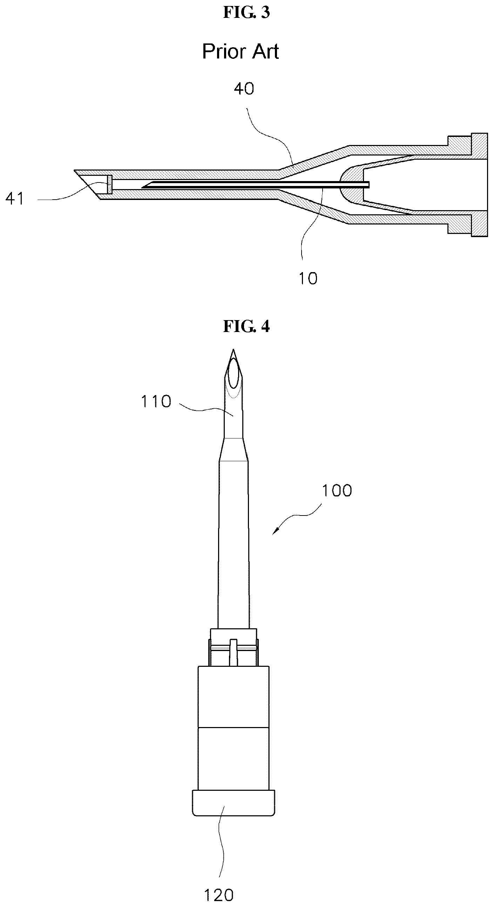

Fig. 2 is an exploded perspective view illustrating a conventional filter-cap syringe, and Fig. 3 is a sectional view illustrating a main portion of the conventional filter-cap syringe, wherein Figs. 2 and 3 illustrate the conventional filter-cap syringe disclosed in US Patent No. 4,180,071.

Since the conventional filter-cap syringe has a cap 40 provided with a filter 41 as shown in Fig. 3 so that foreign substances are filtered by the filter 41 when a liquid medicine is sucked, and the cap 40 including the filter 41 is removed by detaching it from an injection needle 10 upon injection of the liquid medicine, there is no concern that foreign substances may be injected together with the liquid medicine.

However, this conventional filter-cap syringe has limitation on enlargement of the area of a filter means because the diameter of the filter cap should be smaller than that of an inlet of a vial and it is also difficult to form a tip having high strength if the diameter of the filter cap is large.

In addition, since the liquid medicine can be sucked into a cylinder 20 of the syringe only through the minute injection needle 10 after the liquid medicine passes through the filter 41 provided in the cap 40, a relatively large force is required for sucking the liquid medicine and thus there is a problem of considerable inconvenience of use of this syringe.

In other words, the development of the filter syringe in the related art has focused on a point that foreign substances should be filtered at an inlet of the suction flow passage so that a contaminated liquid medicine which has not yet undergone filtration does not contaminate the injection needle.

However, according to the fact that this inventor found in connection with direct manufacture of such a syringe, when the syringe is manufactured, foreign substances produced during the manufacture of the syringe inevitably remain in the interior of the syringe as much as foreign substances such as glass particles which are scattered into and mixed with a liquid medicine in a process of breaking and opening an ampoule having the liquid medicine stored therein.

In order to solve this problem, the filter for filtering foreign substances should not be formed in the suction flow passage for sucking the liquid medicine, but should be formed in an injection flow passage for injecting the liquid medicine into a human body, and at the same time, the injection needle should be isolated so as not to allow a liquid medicine, which has not yet undergone filtration, to contaminate the injection needle.

However, heretofore, there was no injection device satisfying all of these requirements.

That is, the conventional filter syringe has problems in that if the filter is provided in the suction flow passage, foreign substances are filtered upon suction of the liquid medicine but foreign substances remaining within the syringe upon manufacture of the syringe cannot be filtered, and in that if the filter is provided in the injection flow passage, the injection needle is inevitably contaminated by the liquid medicine which has been contaminated upon suction of the liquid medicine.

### [Prior Art Documents]

Korean Patent Laid Open Publication No. 10-2012-0087587
US Patent No. 4180071

### [Disclosure]

### [Technical Problem]

The present invention is conceived to solve the above problems. An object of the present invention is to provide an outer cap of a liquid medicine-injecting device, which is configured such that firm sealing is established between an inner peripheral surface of the outer cap and a peripheral surface of the liquid medicine-injecting device so as to stably generate a negative pressure in a suction flow passage upon suction of a liquid medicine so that the liquid medicine-injecting device having a filter more firmly sucks the liquid medicine, and particularly to prevent the liquid medicine from penetrating an injection needle upon suction of the liquid medicine so that the injection needle is fundamentally prevented from being contaminated by a liquid medicine that has not yet undergone filtration, thereby maximizing marketability and user's safety of the liquid medicine-injecting device having the filter.

### [Technical Solution]

An outer cap of a liquid medicine-injecting device according to the present invention for achieving the object includes a suction needle for sucking a liquid medicine and a cap hub hermetically coupled to the liquid medicine-injecting device, and the outer cap has an internal space extending from the suction needle to the cap hub, wherein the internal space is formed with an injection needle-receiving portion for receiving an injection needle when the liquid medicine-injecting device is assembled; and wherein a sealing means configured to be in contact with a peripheral surface of the liquid medicine-injecting device so as to maintain airtightness between the inner space and the injection needle-receiving portion when the liquid medicine-injecting device is assembled is provided at an inlet of the injection needle-receiving portion.

Here, it is preferable that the sealing means is an hermetic contact end integrally extending from an inner wall of the outer cap and having a section to be brought into contact with the peripheral surface of the liquid medicine-injecting device.

The hermetic contact end is preferably formed with a thin flesh portion opposite to the inner wall of the outer cap and having a thickness smaller than that of the hermetic contact end so that the hermetic contact end may be brought into contact with the peripheral surface of the liquid medicine-injecting device by means of resilient flexibility.

Additionally, an elastic hermetic member may be provided on the hermetic contact end such that the elastic hermetic member is disposed between the hermetic contact end and the peripheral surface of the liquid medicine-injecting device.

Preferably, a plurality of unidirectional inclined wings are formed on an inner peripheral surface of the elastic hermetic member such that they are arranged to be inclined in an assembly direction of the liquid medicine-injecting device, thereby forming a plurality of hermetic structures composed of the unidirectional inclined wings between the elastic hermetic member and the peripheral surface of the liquid medicine-injecting device.

Alternatively, the sealing means may be a ring-shaped elastic hermetic body provided on the inner wall of the outer cap and having a section to be brought into resilient contact with the peripheral surface of the liquid medicine-injecting device.

In this case, it is preferable that a plurality of unidirectional inclined wings are formed on an inner peripheral surface of the ring-shaped elastic hermetic body such that they are arranged to be inclined in an assembly direction of the liquid medicine-injecting device, thereby forming a plurality of hermetic structures composed of the unidirectional inclined wings between the ring-shaped elastic hermetic body and the peripheral surface of the liquid medicine-injecting device.

Additionally, it is most preferable that a control protrusion for controlling opening and closing of the one-way valve means provided in the liquid medicine-injecting device is integrally and protrudingly formed on the inner wall of the outer cap.

### [Advantageous Effects]

According to the invention described above, firm sealing is established between an inner peripheral surface of the outer cap and the peripheral surface of the liquid medicine-injecting device so as to stably generate a negative pressure in a suction flow passage upon suction of the liquid medicine so that the liquid medicine-injecting device having a filter more firmly sucks the liquid medicine, and particularly to prevent the liquid medicine from penetrating the injection needle upon suction of the liquid medicine so that the injection needle is fundamentally prevented from being contaminated by a liquid medicine that has not yet undergone filtration, thereby maximizing marketability and user's safety of the liquid medicine-injecting device having the filter.

### [Description of Drawings]

Fig. 1 is an exploded perspective view illustrating a conventional syringe.
Fig. 2 is an exploded perspective view illustrating a conventional filter-cap syringe.
Fig. 3 is a sectional view illustrating a main portion of the conventional filter-cap syringe.
Fig. 4 is a view illustrating an outer cap of a liquid medicine-injecting device according to the present invention.
Fig. 5 is an exploded view illustrating the outer cap of the liquid medicine-injecting device according to the present invention.
Fig. 6 is a sectional view illustrating a first embodiment of the outer cap of the liquid medicine-injecting device according to the present invention.
Fig. 7 is an exploded sectional view illustrating the first embodiment of the outer cap of the liquid medicine-injecting device according to the present invention.
Fig. 8 is a sectional view illustrating a main portion of a modified example of the first embodiment of the outer cap of the liquid medicine-injecting device according to the present invention.
Fig. 9 is a sectional view illustrating a main portion of another modified example of the first embodiment of the outer cap of the liquid medicine-injecting device according to the present invention.
Fig. 10 is a sectional view illustrating a main portion of a second embodiment of the outer cap of the liquid medicine-injecting device according to the present invention.
Fig. 11 is a sectional view illustrating a main portion of a modified example of the second embodiment of the outer cap of the liquid medicine-injecting device according to the present invention.

### [Best Mode]

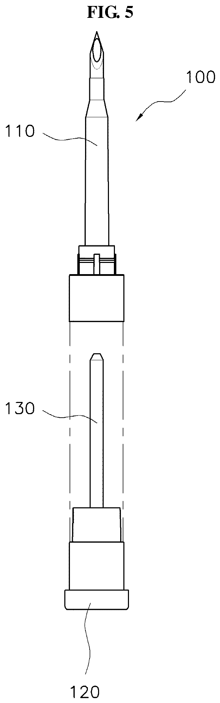

Fig. 4 is a view illustrating an outer cap of a liquid medicine-injecting device according to the present invention, and Fig. 5 is an exploded view illustrating the outer cap of the liquid medicine-injecting device according to the present invention.

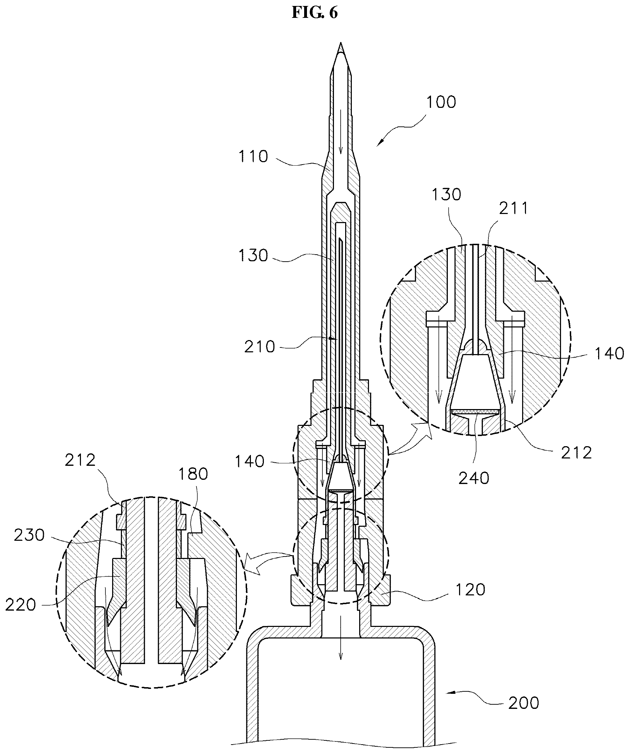

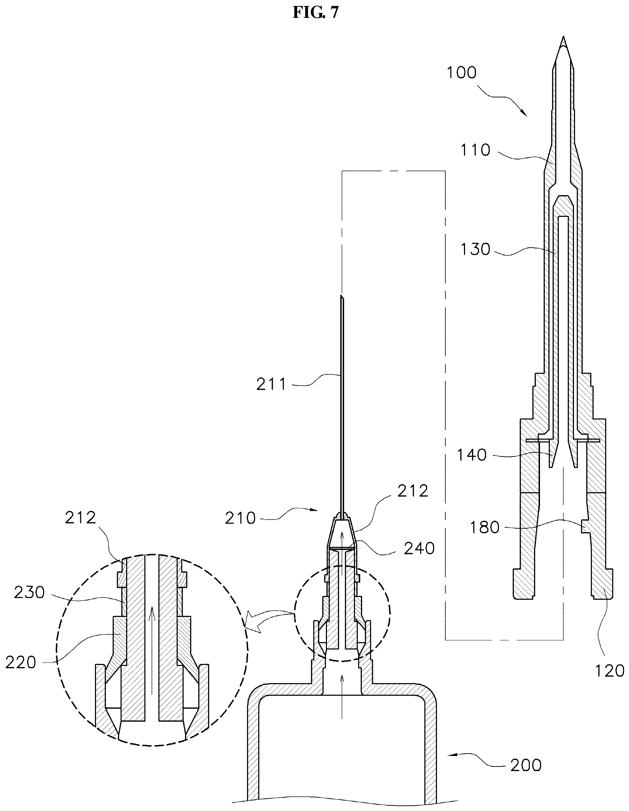

Further, Fig. 6 is a sectional view illustrating a first embodiment of the outer cap of the liquid medicine-injecting device according to the present invention, and Fig. 7 is an exploded sectional view illustrating the first embodiment of the outer cap of the liquid medicine-injecting device according to the present invention.

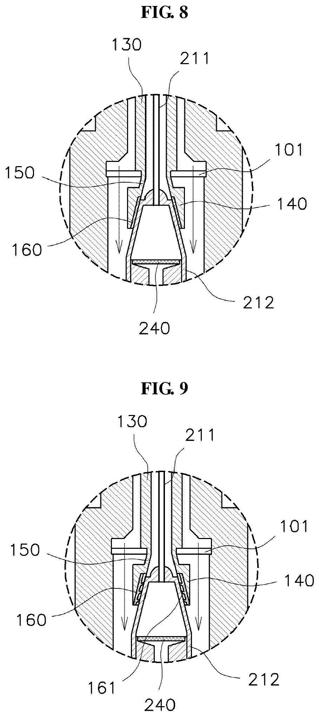

Moreover, Fig. 8 is a sectional view illustrating a main portion of a modified example of the first embodiment of the outer cap of the liquid medicine-injecting device according to the present invention, and Fig. 9 is a sectional view illustrating a main portion of another modified example of the first embodiment of the outer cap of the liquid medicine-injecting device according to the present invention.

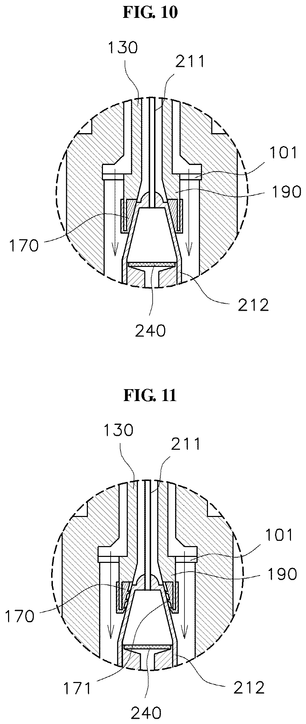

Finally, Fig. 10 is a sectional view illustrating a main portion of a second embodiment of the outer cap of the liquid medicine-injecting device according to the present invention, and Fig. 11 is a sectional view illustrating a main portion of a modified example of the second embodiment of the outer cap of the liquid medicine-injecting device according to the present invention.

As illustrated in Figs. 4 to 11, an outer cap of a liquid medicine-injecting device according to the present invention is configured that firm sealing is established between an inner peripheral surface of the outer cap 100 and a peripheral surface of the liquid medicine-injecting device 200 so as to stably generate a negative pressure in a suction flow passage upon suction of a liquid medicine so that the liquid medicine-injecting device 200 having a filter more firmly sucks the liquid medicine, and particularly to prevent the liquid medicine from penetrating an injection needle 210 upon suction of the liquid medicine so that the injection needle 210 is fundamentally prevented from being contaminated by a liquid medicine that has not yet undergone filtration, thereby maximizing marketability and user's safety of the liquid medicine-injecting device 200 having the filter.

Embodiments of the present invention will be described below in detail with reference to the accompanying drawings.

Hereinafter, for convenience of explanation, all flow passages extending from various types of liquid medicine containers including a vial to the liquid medicine-injecting device 200 coupled with the injection needle 210 will be collectively referred to as the suction flow passage, and all flow passages extending from the liquid medicine-injecting device 200 to the injection needle 210 inserted into a human body will be collectively referred to as an injection flow passage.

Here, the liquid medicine-injecting device 200 includes all of various types of devices, which incorporate a syringe, a connector, a three-way valve and the like and may directly inject a liquid medicine or may be accessorily utilized to inject a liquid medicine.

The outer cap 100 of the liquid medicine-injecting device 200 according to the present invention includes a suction needle 110 for sucking the liquid medicine and a cap hub 120 hermetically coupled to the liquid medicine-injecting device 200, and the outer cap 100 has an internal space extending from the suction needle 110 to the cap hub 120, wherein the internal space is formed with an injection needle-receiving portion 130 for receiving the injection needle 210 when the liquid medicine-injecting device 200 is assembled; and wherein a sealing means configured to be in contact with a peripheral surface of the liquid medicine-injecting device 200 so as to maintain airtightness between the inner space and the injection needle-receiving portion 130 when the liquid medicine-injecting device 200 is assembled is provided at an inlet of the injection needle-receiving portion 130.

First of all, as illustrated in Fig. 4, the outer cap 100 of the liquid medicine-injecting device according to the present invention is basically to suck a liquid medicine from a liquid medicine container and includes the suction needle 110 and the cap hub 120, wherein the suction needle 110 is configured to have an inclined tip so as to pierce a vial, which is one of various packages for liquid medicines, or the like, and is made of a metal material or synthetic resin material to have high strength.

In addition, the cap hub 120 is integrally formed with the suction needle 110 to support the suction needle, and as illustrated in Fig. 6, an inner peripheral surface of the cap hub 120 is engaged with an outer peripheral surface of the liquid medicine-injecting device 200, which is coupled with the injection needle 210, to maintain airtightness.

It is preferable to mold the outer cap 100 as a unitary member. Alternatively, in consideration of moldability due to a complicated sectional structure formed in the outer cap, the outer cap may be manufactured by molding two or more components with a synthetic resin material as illustrated in Fig. 5 and then integrating the components by means of a known bonding or fusing method or the like as illustrated in Fig. 4.

Accordingly, the internal space extending from the suction needle 110 to the cap hub 120 is defined within the outer cap 100, and the internal space of the outer cap 100 will be utilized as the suction flow passage for sucking the liquid medicine into the liquid medicine-injecting device 200.

Particularly, according to the present invention, the injection needle-receiving portion 130 is defined in the internal space of the outer cap 100 as illustrated in Fig. 6.

The injection needle-receiving portion 130 is in the shape of an elongated pipe such that the injection needle 210 may be received in the injection needle-receiving portion, and a distal end of the injection needle-receiving portion is closed, while the inlet of the injection needle-receiving portion is opened to enable the injection needle 210 to enter and exit the injection needle-receiving portion.

That is, when the outer cap 100 is assembled to the liquid medicine-injecting device 200, the injection needle 210 that is located at a front end of the liquid medicine-injecting device 200 to form the injection flow passage is placed within the injection needle-receiving portion 130.

This injection needle-receiving portion 130 is also integrally formed as a part of the outer cap 100, and a bridge 101 is formed to support the injection needle-receiving portion 130 so that the injection needle-receiving portion 130 may be supported without impeding a flow of the liquid medicine.

Additionally, according to the present invention, the sealing means configured to be in contact with the peripheral surface of the liquid medicine-injecting device 200 in response to assembly of the liquid medicine-injecting device 200 so as to maintain airtightness between the inner space and the injection needle-receiving portion 130 is provided at the inlet of the injection needle-receiving portion 130.

That is, since the airtightness between the inner space of the outer cap 100 and the injection needle-receiving portion 130 is maintained by means of the sealing means, the injection needle 210 is prevented from being contaminated by a liquid medicine that has not yet undergone filtration, while the injection flow passage remains in a closed state.

However, when the outer cap 100 is removed, the injection needle 210 forming the injection flow passage is exposed as shown in Fig. 7 to switch the injection flow passage from the closed state to an open state.

The sealing means performing such a function may be a structure formed inside the outer cap 100 or may be a separate component.

Hereinafter, the present invention will be described by way of distinct embodiments: a first embodiment in which the structural sealing means integrally formed inside the outer cap 100 is employed, and a second embodiment in which the sealing means in the form of the separate component is employed inside the outer cap 100.

### [First embodiment]

In the first embodiment of the outer cap of the liquid medicine-injecting device according to the present invention, it is preferable that the sealing means is an hermetic contact end 140, which integrally extends from an inner wall of the outer cap 100 and has a section to be brought into contact with the peripheral surface of the liquid medicine-injecting device 200.

That is, as illustrated in Fig. 6, the sealing means in the first embodiment may consist of the hermetic contact end 140 formed on the inner wall of the outer cap 100.

The hermetic contact end 140 extends from the inner wall of the outer cap 100, more specifically, from a portion at which the injection needle-receiving portion 130 meets the bridge 101, by a certain length to be brought into contact with the peripheral surface of the liquid medicine-injecting device 200.

It will be preferable that in order to enhance the airtightness, the hermetic contact end 140 is brought into surface contact with the liquid medicine-injecting device 200 rather than line contact therewith.

As a result, when the outer cap 100 is assembled to the liquid medicine-injecting device 200, the injection needle 210 is placed in the injection needle-receiving portion 130, while the inlet of the injection needle-receiving portion 130 is maintained in a hermetic state by the hermetic contact end 140 which is in contact with the peripheral surface of the liquid medicine-injecting device 200.

Additionally, in the present invention, it is preferable that the hermetic contact end 140 is formed with a thin flesh portion 150 opposite to the inner wall of the outer cap 100 and having a thickness smaller than that of the hermetic contact end 140 as illustrated in Fig. 8 so that the hermetic contact end 140 may be brought into contact with the peripheral surface of the liquid medicine-injecting device 200 by means of resilient flexibility.

That is, the thin flesh portion 150 is formed on a root side of the hermetic contact end 140, so that the thin flesh portion 150 may be resiliently bent and elastically support the hermetic contact end 140.

Accordingly, the hermetic contact end 140 can be resiliently brought into contact with the peripheral surface of the liquid medicine-injecting device 200, thereby more firmly maintaining the airtightness.

In addition, in the present invention, it is preferable that an elastic hermetic member 160 is provided on the hermetic contact end 140 as illustrated in Fig. 8 such that the elastic hermetic member 160 is disposed between the hermetic contact end 140 and the peripheral surface of the liquid medicine-injecting device 200.

That is, the thin flesh portion 150 is formed to enhance the airtightness as described above, and at the same time, the elastic hermetic member 160 made of rubber or preferably silicone is provided on the hermetic contact end 140, so that firmer airtightness may be maintained between the hermetic contact end 140 and the peripheral surface of the liquid medicine-injecting device 200.

The elastic hermetic member 160 may be separately formed in a ring shape and then installed to be supported by the hermetic contact end 140, or the elastic hermetic member 160 may be separately formed in a cover shape and then installed to cover the hermetic contact end 140 such that the elastic hermetic member is supported by the hermetic contact end. There is no limitation on the shape or assembly structure of the elastic hermetic member.

In particular, in the present invention, it will be most preferable that as illustrated in Fig. 9, a plurality of unidirectional inclined wings 161 are formed on an inner peripheral surface of the elastic hermetic member 160 such that they are arranged to be inclined in an assembly direction of the liquid medicine-injecting device 200, thereby forming a plurality of hermetic structures, which are composed of the unidirectional inclined wings 161, between the elastic hermetic member 160 and the peripheral surface of the liquid medicine-injecting device 200.

Accordingly, by forming the plurality of unidirectional inclined wings 161 on the inner peripheral surface of the elastic hermetic member 160 such that they are inclined in an upward direction on the figure, which is the assembly direction of the liquid medicine-injecting device 200, when the outer cap 100 is assembled to the liquid medicine-injecting device 200, tips of the plurality of unidirectional inclined wings 161 come into contact with the peripheral surface of the liquid medicine-injecting device 200, thereby forming the plurality of the hermetic structures.

Since the unidirectional inclined wings 161 configured as described above are deformed to be in closer contact with the peripheral surface of the liquid medicine-injecting device 200 when a negative pressure acts on the suction flow passage, the hermetic structures having high waterproofness can be firmly formed between the hermetic contact end 140 and the peripheral surface of the liquid medicine-injecting device 200.

### [Second embodiment]

In a second embodiment of the outer cap of the liquid medicine-injecting device according to the present invention, it is preferable that as illustrated in Fig. 10, the sealing means is a ring-shaped elastic hermetic body 170 provided on the inner wall of the outer cap 100 and having a section that is to be brought into resilient contact with the peripheral surface of the liquid medicine-injecting device 200.

That is, the second embodiment of the present invention is different from the aforementioned first embodiment in that the hermetic contact end 140 integrally extending from the inner wall of the outer cap 100 is formed in the first embodiment, whereas the ring-shaped elastic hermetic body 170 is configured as a separate component in the second embodiment.

Of course, a configuration for fixing the ring-shaped elastic hermetic body 170 to the outer cap 100 would be required.

For example, a separate support protrusion 190 may be formed to extend from the outer cap 100 to a desired position, and the ring-shaped elastic hermetic body 170 may be then fixed to the support protrusion 190.

It will be apparent that there may be various variations of the outer cap 100 for fixing the ring-shaped elastic hermetic body 170.

Particularly, in the present invention, it is most preferable that as illustrated in Fig. 11, a plurality of unidirectional inclined wings 171 are formed on an inner peripheral surface of the ring-shaped elastic hermetic body 170 such that they are arranged to be inclined in the assembly direction of the liquid medicine-injecting device 200, thereby forming a plurality of hermetic structures, which are composed of the unidirectional inclined wings 171, between the ring-shaped elastic hermetic body 170 and the peripheral surface of the liquid medicine-injecting device 200.

Accordingly, by forming the plurality of unidirectional inclined wings 171 on the inner peripheral surface of the ring-shaped elastic hermetic body 170 such that they are inclined in an upward direction on the figure, which is the assembly direction of the liquid medicine-injecting device 200, when the outer cap 100 is assembled to the liquid medicine-injecting device 200, tips of the plurality of unidirectional inclined wings 171 come into contact with the peripheral surface of the liquid medicine-injecting device 200, thereby forming the plurality of the hermetic structures.

Since the unidirectional inclined wings 171 configured as described above are deformed to be in closer contact with the peripheral surface of the liquid medicine-injecting device 200 when a negative pressure acts on the suction flow passage, the hermetic structures having high waterproofness can be firmly formed between the ring-shaped elastic hermetic body 170 and the peripheral surface of the liquid medicine-injecting device 200.

Although description has been made with regard to the configuration and operation in which the hermetic contact end 140 or the ring-shaped elastic hermetic body 170 as the sealing means is provided in the aforementioned outer cap 100 according to the present invention so that the injection needle 210 is isolated from the suction flow passage and the injection needle 210 is maintained in a closed state, the entire operation of the liquid medicine-injecting device 200 together with the outer cap 100 will be described below.

Here, as described above, the liquid medicine-injecting device 200 includes all of various types of devices, which incorporate a syringe, a connector, a three-way valve and the like and may directly inject a liquid medicine or may be accessorily utilized to inject a liquid medicine.

Hereinafter, for convenience of explanation, the liquid medicine-injecting device 200 including the injection needle 210 composed of a needle body 211 and a hub 212, a one-way valve means 220 for enabling the liquid medicine to flow only in one direction, a fixing ring 230 for preventing detachment of the one-way valve means 220, and a filter 240 for filtering foreign substances from the liquid medicine in the injection flow passage will be described by way of example.

That is, in the present invention, upon suction of the liquid medicine, the one-way valve means 220 in the suction flow passage is deformed and opened as shown in Fig. 6 and the liquid medicine from which foreign substances are not filtered is then sucked into the liquid medicine-injecting device 200. During the suction of the liquid medicine, the hermetic contact end 140 or the ring-shaped elastic hermetic body 170, which is the sealing means of the present invention, closes the injection needle 210 located in the injection flow passage.

On the contrary, upon injection of the liquid medicine, the outer cap 100 is removed to open the injection needle 210 located in the injection flow passage as illustrated in Fig. 7, whereas the suction flow passage is closed by the one-way valve means 220.

According to embodiments, the opening and closing of the one-way valve means 220 may be controlled by itself depending on an acting direction of pressure generated in the liquid medicine-injecting device 200 or may be controlled simply depending on whether the outer cap 100 is detached.

In this regard, since Korean Patent No. 10-1563723 commonly assigned to the applicant of this application discloses a configuration in which opening and closing of such a valve means are controlled depending on whether a cap is detached, a detailed description thereof will be omitted.

Particularly, in the present invention, it is preferable that a control protrusion 180 for controlling the opening and closing of the one-way valve means 220 provided in the liquid medicine-injecting device 200 is integrally and protrudingly formed on the inner wall of the outer cap 100 so that the opening and closing of the one-way valve means can be adjusted by itself depending on the acting direction of the pressure generated in the liquid medicine-injecting device 200 and simultaneously can be adjusted depending on whether the outer cap 100 is detached.

For example, if the control protrusion 180 is not formed, the one-way valve means 220 is opened and closed only depending on the acting direction of the pressure from the liquid medicine-injecting device 200. The opening and closing are not adjusted depending on whether the outer cap 100 is detached.

However, if the control protrusion 180 is formed, the control protrusion 180 causes the deformation of the one-way valve means 220 when the outer cap 100 is assembled, so that the one-way valve means 220 always remains in an open state in the state where the outer cap 100 is assembled.

Accordingly, there is an advantage that convenient use can be achieved even in a case where the liquid medicine has been sucked when the outer cap 100 has been assembled and is then discharged as it is, such as a case where the liquid medicine has been sucked and is not injected directly into a human body, for example, even in a case where a saline solution has been sucked and is then injected into a liquid medicine container accommodating a powder medicine to dissolve the powder medicine in the saline solution and subsequently a mixture of the powder medicine and the saline solution is sucked again.

Meanwhile, since a conventional one-way valve means 220 was made of an elastic material, there was a case where the one-way valve means 220 did not withstand pressure acting on a filter 240 upon injection of a liquid medicine and consequently was detached.

In order to prevent such detachment of the one-way valve means, it is possible to firmly secure the one-way valve means 220 to the liquid medicine-injecting device 200 by using the fixing ring 230.

Moreover, the injection needle 210 is coupled to the front end of the liquid medicine-injecting device 200, and the filter 240 capable of filtering various kinds of foreign substances including minute glass particles contained in the liquid medicine is provided inside the injection needle 210.

Accordingly, upon injection of the liquid medicine, the liquid medicine that has passed through the one-way valve means 220 is subjected to filtration of the foreign substances by the filter 240 and then injected into a human body through the injection needle 210.

Of course, it is also possible to add a additional filter in the suction flow passage, if necessary.

As a result, not only foreign substances contained in the liquid medicine but also foreign substances remaining inside the liquid medicine-injecting device 200 in a manufacturing process can be filtered, and the filtered liquid medicine can be then safely injected.

With this configuration, the suction flow passage of the liquid medicine starts from the liquid medicine container and reaches the liquid medicine-injecting device 200 through the suction needle 110 of the outer cap 100 and the one-way valve means 220.

On the contrary, the injection flow passage starts from the liquid medicine-injecting device 200 and reaches the injection needle 210 through the filter 240.

As for the outer cap of the liquid medicine-injecting device according to the present invention described above, the opening and closing of the one-way valve means 220 depends on whether the outer cap 100 is assembled, wherein when a negative pressure acts on the liquid medicine-injecting device 200, the one-way valve means 220 is opened as illustrated in Fig. 6 to perform suction of the liquid medicine.

On the contrary, when a positive pressure acts on the liquid medicine-injecting device 200 in a state where the outer cap 100 is simply detached upon injection of the liquid medicine, the one-way valve means 220 is closed as shown in Fig. 7, so that the liquid medicine passes through the filter 240 to filter foreign substances and is then injected through the injection needle 210.

Accordingly, the outer cap 100 of the liquid medicine-injecting device according to the present invention has advantages in that firm sealing is established between the inner peripheral surface of the outer cap 100 and the peripheral surface of the liquid medicine-injecting device 200 so as to stably generate a negative pressure in the suction flow passage upon suction of the liquid medicine so that the liquid medicine-injecting device 200 having the filter 240 more firmly sucks the liquid medicine, and particularly to prevent the liquid medicine from penetrating the injection needle 210 upon suction of the liquid medicine so that the injection needle 210 is fundamentally prevented from being contaminated by a liquid medicine that has not yet undergone filtration, thereby maximizing marketability and user's safety of the liquid medicine-injecting device having the filter 240.

Particularly, since the liquid medicine can be injected only by sucking the liquid medicine in the state where the outer cap 100 is assembled and then detaching the outer cap 100, there is also an advantage in that user's convenience can be greatly improved.

Moreover, if the control protrusion 180 is further formed, there is another advantage in that the liquid medicine can be rapidly discharged without passing through the filter 240 even in a state where the outer cap 100 is not detached.

The aforementioned embodiments are merely examples for specifically explaining the spirit of the present invention, and the scope of the present invention is not limited to the figures and embodiments.

**[Explanation of Reference Numerals]**

| | |
|---|---|
| 100: Outer cap | 101: Bridge |
| 110: Suction needle | 120: Cap hub |
| 130: Injection needle-receiving portion | 140: Hermetic contact end |
| 150: Thin flesh portion | 160: Elastic hermetic member |
| 161: Unidirectional inclined wing | 170: Ring-shaped elastic hermetic body |
| 171: Unidirectional inclined wing | 180: Control protrusion |
| 190: Support protrusion | 200: Liquid medicine-injecting device |
| 210: Injection needle | 211: Needle body |
| 212: Hub | 220: One-way valve means |
| 230: Fixing ring | 240: Filter |

## Claims

1. An outer cap of a liquid medicine-injecting device, the outer cap comprising a suction needle for sucking a liquid medicine and a cap hub hermetically coupled to the liquid medicine-injecting device, the outer cap having an internal space extending from the suction needle to the cap hub,
wherein the internal space is formed with an injection needle-receiving portion for receiving an injection needle when the liquid medicine-injecting device is assembled; and
wherein a sealing means configured to be in contact with a peripheral surface of the liquid medicine-injecting device so as to maintain airtightness between the inner space and the injection needle-receiving portion when the liquid medicine-injecting device is assembled is provided at an inlet of the injection needle-receiving portion.

2. The outer cap of the liquid medicine-injecting device of Claim 1, wherein the sealing means is an hermetic contact end integrally extending from an inner wall of the outer cap and having a section to be brought into contact with the peripheral surface of the liquid medicine-injecting device.

3. The outer cap of the liquid medicine-injecting device of Claim 2, wherein the hermetic contact end is formed with a thin flesh portion opposite to the inner wall of the outer cap and having a thickness smaller than that of the hermetic contact end so that the hermetic contact end may be brought into contact with the peripheral surface of the liquid medicine-injecting device by means of resilient flexibility.

4. The outer cap of the liquid medicine-injecting device of Claim 3, wherein an elastic hermetic member is provided on the hermetic contact end such that the elastic hermetic member is disposed between the hermetic contact end and the peripheral surface of the liquid medicine-injecting device.

5. The outer cap of the liquid medicine-injecting device of Claim 4, wherein a plurality of unidirectional inclined wings are formed on an inner peripheral surface of the elastic hermetic member such that they are arranged to be inclined in an assembly direction of the liquid medicine-injecting device, thereby forming a plurality of hermetic structures composed of the unidirectional inclined wings between the elastic hermetic member and the peripheral surface of the liquid medicine-injecting device.

6. The outer cap of the liquid medicine-injecting device of Claim 1, wherein the sealing means is a ring-shaped elastic hermetic body provided on the inner wall of the outer cap and having a section to be brought into resilient contact with the peripheral surface of the liquid medicine-injecting device.

7. The outer cap of the liquid medicine-injecting device of Claim 6, wherein a plurality of unidirectional inclined wings are formed on an inner peripheral surface of the ring-shaped elastic hermetic body such that they are arranged to be inclined in an assembly direction of the liquid medicine-injecting device, thereby forming a plurality of hermetic structures composed of the unidirectional inclined wings between the ring-shaped elastic hermetic body and the peripheral surface of the liquid medicine-injecting device.

8. The outer cap of the liquid medicine-injecting device of Claim 5 or Claim 7, wherein a control protrusion for controlling opening and closing of the one-way valve means provided in the liquid medicine-injecting device is integrally and protrudingly formed on the inner wall of the outer cap.
